Europäisches Patentamt

European Patent Office

Office européen des brevets

(11) Publication number: **0 453 286 A2**

# (12) EUROPEAN PATENT APPLICATION

(21) Application number: 91303473.2

(22) Date of filing: 18.04.91

(51) Int. Cl.⁵: **C08G 18/48, C08K 5/15,**
C08K 7/02, // A61L15/00 ,
(C08G18/48, 101:00)

(30) Priority: 18.04.90 US 510646

(43) Date of publication of application:
23.10.91 Bulletin 91/43

(84) Designated Contracting States:
BE DE ES FR GB IT NL

(71) Applicant: ARCO CHEMICAL TECHNOLOGY
INC.
3 Christina Centre Suite 902 201 N Walnut
Street
Wilmington Delaware 19801 (US)

(72) Inventor: Le-Khac, Bi
1210 Birmingham Road
West Chester, Pennsylvania 19382 (US)
Inventor: Younes, Usama E.
C27 Hollybrook
Newtown Square, Pennsylvania 19073 (US)

(74) Representative: Cropp, John Anthony David et
al
MATHYS & SQUIRE 10 Fleet Street
London, EC4Y 1AY (GB)

(54) Superabsorbent foam composition.

(57) Superabsorbent foams, such as polyurethane or polyisocyanurate resins, may be produced by incorporating superabsorbent materials, such as fibers or powders, into a conventional foam formulation, optionally in the presence of alkylene carbonate, such as propylene carbonate. The superabsorbent polyurethanes may be used in conventional foam applications, such as sponges, personal hygiene products, diapers, etc., where high absorbency of liquids is desired. Particularly suitable superabsorbent materials are Fibersorb® SA Superabsorbent Polymers, which include the compositions of: (a) an aqueous polymer containing at least 25 mole percent recurring units of an $\alpha,\beta$-unsaturated monomer having in its molecule at least one or two carboxyl groups or at least one or two other groups convertible to carboxyl groups, with (b) from about 0.1 to about 10 total parts by weight of at least one reactive compound per 100 parts by weight of the partially neutralized aqueous polymer composition, the reactive compound being a water soluble compound bearing a reactive group from the group consisting of an amine group, a hydroxyl group and mixtures thereof.

EP 0 453 286 A2

## Field of the Invention

The invention relates to the synthesis of polyurethane compositions and methods for making the same, and in one aspect, is more particularly related to polyurethane compositions having enhanced absorbency properties.

## Background of the Invention

Polyurethane foams, formed by the reaction of a polyisocyanate with a polyhydroxyl-containing compound in the presence of a suitable catalyst, are widely accepted as padding materials for cushions in furniture, automobiles and the like. Polyurethane foams are also used in sponges and for other uses that require high liquid absorption properties, such as specialty packaging and personal care and hygiene items, including highly absorbent diapers. Polyisocyanurate foams, made with a different catalyst to increase cross-linking relative to polyurethane foams, are often used as insulation in the building and construction industry.

Non-polyurethane water absorbent polymer compositions are known in the art. For example, U.S. Pat. No. 4,705,773 teaches a water-insoluble, water-swellable polymer composition comprising a copolymer of styrene and maleic anhydride and a multi-arm block copolymer of styrene and ethylene oxide. Sugar-containing water-absorbing compositions which facilitate fiber formation include copolymers of recurring units of at least one $\alpha,\beta$-unsaturated monomer and recurring units of at least one copolymerizable comonomer comprising from about 20 to about 80 percent pendant carboxylic acid units and from about 80 to about 20 percent pendant carboxylate salt units, blended with at least one disaccharide or oligosaccharide, according to U.S. Pat. No. 4,788,237. U.S. Pat. No. 4,731,067 discusses a copolymer of recurring units of at least one $\alpha,\beta$-unsaturated monomer and recurring units of at least one copolymerizable comonomer comprising, from about 20 to about 80 percent pendant carboxylic acid units and from about 80 to 20 percent pendant carboxylate salt units, which is blended with at least one monomer containing at least two hydroxyl groups to produce a water-absorbing composition upon curing. See also U.S. pat Nos. 4,743,244; 4,813,945 and 4,880,868 which relate to other superabsorbing polymers.

However, it would be useful to devise an improved polyurethane composition having improved, liquid-absorbing properties.

Accordingly, it is an object of the present invention to provide a polyurethane composition that has improved liquid-absorbing properties.

It is another object of the present invention to provide a superabsorbent polyurethane composition that can be readily produced with available polyurethane technology.

It is yet another object of the invention to provide a superabsorbent polyurethane composition that is otherwise similar to polyurethane compositions of conventional absorbancy and which may have similar densities thereto.

In carrying out these and other objects of the invention, there is provided, in one form, a superabsorbent foam comprising the reaction product of a polyol with a polyisocyanate in the presence of a foam catalyst, and further in the presence of a polymeric superabsorbent material.

The term "superabsorbent" as used in the phrase "polymeric superabsorbent material" is intended to distinguish these materials from conventional absorbent materials such as cotton which may absorb up to about 30% of their own weight of liquid by physical adsorption on the surface. By contrast, polymeric superabsorbent materials are capable of absorbing significantly greater quantities of liquid, e.g. up to as much as many times their own weight, and swell as part of their adsorption mechanism.

It has been discovered that superabsorbent fibers or powders may be readily incorporated into foam compositions, such as polyurethane and polyisocyanurate resins, to produce superabsorbent foam compositions, such as flexible and rigid foams.

The superabsorbent materials, which may be in a number of forms, including, but not limited to fibers and powders, are known to be highly absorbent of liquids themselves. Generally, these materials are organic/inorganic copolymers, and a particularly preferred group of superabsorbent materials are the Fibersorb® SA Superabsorbent polymers manufactured by ARCO Chemical Company. These materials may be more specifically identified as fibers or powders cured from a stable, heat curable aqueous composition having: (a) an aqueous polymer composition containing at least 25 mole percent recurring units of an $\alpha,\beta$ unsaturated monomer having in its molecule at least one or two carboxyl groups or at least one or two other groups convertible to carboxyl groups, with (b) from about 0.1 to about 10 total parts by weight of at least one reactive compound per 100 parts by weight of the partially neutralized aqueous polymer composition, the reactive compound being a water soluble compound bearing a reactive group from the group consisting of an amine group, a hydroxyl group and mixtures thereof.

More specifically, the superabsorbent fibers or powders can be defined as being cured from a stable, heat curable aqueous composition having: (a) a partially neutralized aqueous polymer composition prepared by the reaction of a base with a polymer containing at least 25 mole percent recurring units of an $\alpha,\beta$-unsaturated monomer having in its molecule at least one or two carboxyl groups or at least one or two other groups convertible to carboxyl groups, the degree of neutralization of said partially neutralized polymer being within the range of from about 0.2 to about 0.8 equivalent of total carboxyl groups or groups convertible to carboxyl groups of the $\alpha,\beta$-unsaturated monomer, with (b) from about 0.1 to about 10 total parts by weight of at least one reactive compound per 100 parts by weight of the partially neutralized aqueous polymer composition, the reactive compound being a water soluble compound bearing a reactive group from the group consisting of an amine group, a hydroxyl group and mixtures thereof.

When the reactive compound contains an amine group, a reaction product is formed as the curable aqueous composition. The reaction product which is formed by substituted ammonium carboxylate ionic bonding between the unneutralized carboxyl groups on the polymer and the amine groups on the reactive compound is stable at room temperature and can be made into absorbent fibers through curing with heat. When the reactive compound does not contain an amine group, then a reaction product does not form, though the resultant curable, stable aqueous composition may also be cured upon the application of heat to give absorbent fibers or powders. The term "curable aqueous composition" encompasses both the instance where the reactive compound is an amine and a reaction product is formed, and the case where amine groups are not present on the reactive compound and no reaction occurs, nevertheless a stable, heat curable blend of materials is created. The base used for neutralization may be a strong base.

This stable liquid "syrup" of this aqueous composition is cured by heating and driving off the water. The reactive compound may contain at least two reactive hydrogens, for example, and may have at least two hydroxyl groups, two primary amine groups or at least one hydroxyl group and at least one primary amine group.

The polymer mentioned in the material (a) of the stable, heat curable aqueous composition may include, but is not limited to $\alpha$-olefin/maleic anhydride copolymers, $\alpha$-olefin/citraconic anhydride copolymers, $\alpha$-olefin/acrylic acid copolymers, $\alpha$-olefin/methacrylic acid copolymers, vinyl compound/maleic anhydride copolymers, vinyl compound/citraconic anhydride copolymers, vinyl compound/acrylic anhydride copolymers, vinyl compound/methacrylic acid copolymers, alkyl acrylate/maleic anhydride copolymers, alkyl acrylate/citraconic anhydride copolymers, alkyl vinyl ether/maleic anhydride copolymers, alkyl vinyl ether/citraconic anhydride copolymers, $\alpha$-olefin/maleic anhydride/$\alpha$-olefin terpolymers, and vinyl acetate/maleic anhydride copolymers, as representative examples only.

Other useful unsaturated monomers which may be used to form a homopolymer or a copolymer with other such monomers or the copolymers mentioned above include, but are not limited to, maleic acid; crotonic acid; fumaric acid; mesaconic acid; the sodium salt of maleic acid; the sodium salt of 2-methyl-2-butene dicarboxylic acid; the sodium salt of itaconic acid; maleamic acid; maleamide; N-phenyl maleimide; maleimide; maleic anhydride (as noted); fumaric anhydride; itaconic anhydride; citraconic anhydride; mesaconic anhydride; methyl itaconic anhydride; ethyl maleic anhydride; diethylmaleate; methylmaleate; ethylene, propylene, isobutylene, $C_1$ to $C_4$ alkyl methacrylates, vinyl acetate, methyl vinyl ether, styrenic compounds and the like, and their mixtures.

Specific reactive compounds contemplated in material (b) of the stable, heat curable aqueous composition may include, but are not limited to ethanolamine; *tris*-(hydroxymethyl)aminomethane; 3-amino-1-propanol; DL-1-amino-2-propanol; 2-amino-1-butanol; N-N-dimethylethanolamine; diisopropanolamine; methyldiethanolamine; triethanolamine; 2-(methylamino)ethanol; and the like and mixtures thereof.

Other reactive compounds in the definition above include, but are not limited to, compounds having at least two hydroxyl groups, such as diols; compounds having at least two amine groups, such as diamines; aminoalcohols having at least one of each and mixtures thereof. Specific examples include, but are not limited to, polyethers, such as poly(ethylene oxide), poly(propylene oxide), poly(isobutylene oxide), poly(tetramethylene oxide), and random and block linear or branched polyether copolymers of various oxides; hydroxyl-containing compounds such as ethylene glycol, propylene glycol, trimethylene glycol, 1,3-propane diol, neopentyl glycol, 1,5-pentane diol, diethylene glycol, dipropylene glycol, 1,4-cyclohexane dimethanol, Bisphenol A, 1,4-*bis*-($\beta$-hydroxyethoxy)*bis*-phenol, hydroquinone, phloroglucinol, glycerol, erythritol, pentaerythritol, 1,7-dihydroxylsedoheptulose, and the like; saccharides, such as disaccharides and oligosaccharides; even multi arm block copolymers of styrene and alkylene oxides, such as ethylene oxide, and the like.

The resultant fibers or powders are known to be very absorbent or "super"-absorbent of liquids including, but not limited to such liquids as aqueous solutions, such as water; alcohols; and the like.

It is anticipated that a wide variety of materials may be used to enhance the liquid absorbency properties of polyurethane materials within the broad description given above, including, but not necessarily limited to the materials described in U.S. Pat Nos. 4,705,773; 4,731,067; 4,743,244; 4,788,237; 4,813,945 and 4,880,868 to Bi Le-Khac and assigned to ARCO Chemical Company, incorporated by reference herein. More details about the methods of making the superabsorbing materials used herein and their structures may be found in these various patents. Materials known under the trade name Lanseal, produced by Nichon Exlan, Japan, may also be suitable.

It is anticipated that the superabsorbent materials may be readily incorporated into most conventional polyurethane formulations with few, if any, changes to the formulation. The superabsorbent materials are simply added to the formulation as one of the components and are not prereacted with any particular component before addition, although this method of addition is not necessarily precluded herein. However, it will be appreciated that simply adding them to the formulation is a preferred method since it is readily integrated into existing foaming procedures. It is expected that the other properties of the foam will not be adversely affected by the use of the superabsorbent materials. Broadly, up to 25 parts per hundred (pph), based on the total composition weight, are expected to be useful, with up to 20 pph being preferred in one aspect of the invention. An even more preferred range is from about 5 to about 10 pph of the superabsorbent materials.

In accordance with this invention, the remaining polyurethane foam components are expected to be conventional, indicating that the invention is compatible with standard formulations. For example, a variety of polyether and/or polyester polyols are expected to be useful in making the polyurethane compositions of this invention. These polyols include, but are not necessarily limited to ethylene oxide (EO) capped polyols and polyols not capped with EO, as well as propylene oxide (PO) and/or butylene oxide containing polyols. The polyols may contain blocks or random distributions of these various alkylene oxides added to suitable initiators. In one aspect, the polyol preferably has a molecular weight of from about 300 to 10,000, and is typically made by the reaction of an initiator having a plurality of reactive hydrogens thereon with one or more alkylene oxides. Suitable initiators include, but are not limited to , glycerin, alkanolamines, alkylamines, aryl or aromatic amines, sucrose, sorbitol, trimethylol propane (TMP), $\alpha$-methylglucoside, $\beta$-methylglucoside, or other methylglucoside, resins of phenol, aniline and mixed phenol aniline, such as methylenedianiline or bisphenol A, Mannich condensates and mixtures thereof, for example. The polyol may be made by alkoxylating the initiator with a desired number of moles of an alkylene oxide. Preferably, the alkylene oxide has from two to four carbon atoms, and is thus, EO, PO, butylene oxide or mixtures thereof, as noted. The oxides may be mixed upon addition, or may be added to the polyol initiator chain separately to form blocks or caps. In one aspect, a mixture of ethylene oxide and propylene oxide is added to the initiator. The alkoxylation may or may not be catalyzed; KOH is a commonly used catalyst, although others may be employed. For example, double metal cyanide catalysts may be employed, in particular zinc hexacyanocobaltate, and the polyols may be prepared in accordance with the methods described in U.S. Pat. Nos. 3,029, 505; 3,900,518; 3,941,049 and 4,355,100, incorporated by reference herein. Alternatively, various polymer polyols may also be employed as completely replacing or in conjunction with suitable polyol components.

A catalyst is typically employed in preparing polyurethane and polyisocyanurate foams in the conventional manner. Generally, catalysts to make rigid polyisocyanurate foams provide more cross-linking than do catalysts used to make more flexible polyurethane foams. Such catalysts may include one or more of the following:

(a) Tertiary amines such as trimethylamine; triethylamine; N-methylmorpholine; N-ethylmorpholine; N,N-dimethylbenzylamine; N,N-dimethylethanolamine; N,N,N′,N′-tetramethyl-1,3-butanediamine; N,N-dimethylpiperazine; 1,4-diazobicyclo [2.2.2]octane and the like;

(b) Tertiary phosphines such as trialkylphosphines; dialkylbenzylphosphines, and the like;

(c) Strong bases, such as alkali and alkaline earth metal hydroxides; alkoxides; and phenoxides;

(d) Acidic metal salts of strong acids, such as ferric chloride; stannic chloride; stannous chloride; antimony trichloride; bismuth nitrate and chloride; and the like;

(e) Chelates of various metals such as those which can be obtained from acetylacetone; benzoylacetone; trifluoroacetyl acetone; ethyl acetoacetate; salicylaldehyde; cyclopentanone-1-carboxylate; acetylacetoimine; bis-acetylacetonealkylenediamine; salicylaldehydeimine; and the like, with various metals such as Be, Mg, Zn, Cd, Pd, Ti, Zr, Sn, As, Bi, Cr, Mo, Mn, Fe, Co, and Ni;

(f) Alcoholates and phenolates of various metals, such as $Ti(OR)_4$; $Sn(OR)_4$; $Al(OR)_3$; and the like, wherein R is alkyl or aryl, and the reaction products of alcoholates with carboxylic acids, $\beta$-diketones, and 2-(N,N-dialkylamino)alcohols;

(g) Salts of organic acids with a variety of metals, such as alkali metals, alkaline earth metals, Al, Sn, Pb, Mn, Co, Ni and Cu, including, for example, sodium acetate, stannous octoate, stannous oleate, lead octoate, metallic driers such as manganese and cobalt naphthenate, and the like; and

(h) Organometallic derivatives of tetravalent tin, trivalent and pentavalent As, Sb, and Bi and metal carbonyls of iron and cobalt.

Of course, combinations of any of the above polyurethane catalysts may be employed. Usually, the amount of catalyst employed ranges from about 0.01 to about 5.0 pbw percent, based on 100 pbw of the polyol. More often, the amount of the catalyst used is about 0.2 to about 2.0 pbw.

The polyol component for the polyurethane composition is reacted in the presence of one or more of the above catalysts with a polyisocyanate according to conventional procedures. The polyisocyanate used may be any aromatic or aliphatic polyisocyanate, such as toluene diisocyanates (TDIs); polymeric isocyanates and aliphatic diisocyanates. Typical aromatic polyisocyanates include, but are not limited to, *m*-phenylene diisocyanate; *p*-phenylene diisocyanate; polymethylene polyphenylisocyanate; 2,4-toluene diisocyanate; 2,6-toluene diisocyanate; dianisidine diisocyanate; bitolylene diisocyanate; naphthalene-1,4-diisocyanate; diphenylene-4,4'-diisocyanate; aliphatic-aromatic diisocyanates, such as xylylene- 1,4-diisocyanate; xylylene-1,2-diisocyanate; xylylene-1,3-diisocyanate; *bis*(4-isocyanatophenyl)methane; *bis*(3-methyl-4-isocyanatophenyl)methane; and 4,4'-diphenylpropane diisocyanate. Suitable aliphatic diisocyanates would include isophorone diisocyanate; hexamethylene diisocyanate; and methylene-*bis*-cyclohexylisocyanate. Toluene diisocyanates are preferred, in one aspect of the invention.

Aromatic polyisocyanates suitable for use include methylene-bridged polyphenyl polyisocyanate mixtures which have a functionality of from about 2 to about 4. These latter isocyanate compounds are generally produced by the phosgenation of corresponding methylene bridged polyphenyl polyamines, which are conventionally produced by the reaction of formaldehyde and primary aromatic amines, such as aniline in the presence of hydrochloric acid and/or other acidic catalysts.

Foaming is carried out in the presence of water and, optionally, additional organic blowing agents. The water is normally used in amounts of 0.5 to 15 parts by weight, preferably, 1.0 to 10 pbw based on 100 pbw of the polyol present. The organic blowing agents optionally used along with the water are known in the art and may include, but are not limited to, monofluorotrichloromethane, difluorodichloromethane, methylene dichlorie and others generally known in the art. Additives to regulate the cell size and the cell structure, for example silicone surfactants such as dimethylpolysilioxanes, may also be added to the foaming mixture. Fillers, dyes or plasticizers of known types may also be used, of course. These and other additives are well known to those skilled in the art.

It has been additionally discovered that the use of propylene carbonate in the mixture permits the mixture to have a lower viscosity and allows better handling of the superabsorbent material in the polyol. The propylene carbonate could be provided in a form mixed with a catalyst such as ARSET® catalyst, a propylene carbonate/amine catalyst mixture produced by ARCO Chemical Company. Other alkylene carbonates, such as ethylene carbonate, are also expected to be useful herein. In one aspect of the invention, from 2 to 50 pbw of an alkylene carbonate may be used, with 5 to 20 pbw being a narrower range applicable under certain conditions.

It is to be understood that the polymeric superabsorbent material may be incorporated into the foam-forming reaction mixture in fully cured or not fully cured, e.g. uncured form.

The method and superabsorbent polyurethanes of the invention will be further described with respect to the following illustrative examples. The Fibersorb material used in all Examples is Fibersorb SA 7000.

EXAMPLES 1 -3

The following formulations were mixed and poured into a foaming box 10 x 5 x 5 inches. All units are in grams unless otherwise noted.

| Example | 1 (Control) | 2 | 3 |
|---|---|---|---|
| SF 5505 Polyol | 100 | 100 | 100 |
| L6202 Silicone | 1.1 | 1.1 | 1.1 |
| Amine 1/2/3 Catalyst | 0.21 | 0.21 | 0.21 |
| Water | 4.0 | 4.0 | 4.0 |
| T-10 Catalyst | 0.4 | 0.4 | 0.4 |
| Propylene Carbonate | 10.0 | 10.0 | 10.0 |
| Fibersorb® SA 7000 Superabsorbent Fiber(1/8" long fibers, pph) | 0 parts/100 | 3.0 parts/100 | 6.1 parts/100 |
| TDI | 48.9 | 48.9 | 48.9 |
| Foam Density (pcf) | 2.01 | 2.1 | 2.2 |

5

Each of the foams took about 30 seconds to rise after which each was postcured at 120°C. for five minutes. The resulting foams had comparable densities.

Each of the foams was then soaked with deionized water. Then the amount of water retention was measured after a force of 1.2 psi was applied for one minute and the retention after a full squeeze was measured. The results of the water absorbency are shown in the following Table 1.

## TABLE I
### Water Absorbency Results

| Example | 1 (Control) | 2 | 3 |
|---|---|---|---|
| Free Swell (g/g) | 21.8 | 22.2 | 19.8 |
| Retention, g/g at 1.2 psi. | 5.2 | 7.3 | 9.7 |
| Full Squeeze Retention, >80 psi (g/g) | 1.9 | 4.6 | 8.0 |

The results of the experiments indicate that it is possible to blend superabsorbent fibers into urethane foam formulations in the presence of propylene carbonate to obtain excellent quality polyurethane foams that have the ability to retain water and other liquids to a considerably greater degree than foams without such fibers, and thus can be used as a superabsorbent polyurethane sponge or the like.

### EXAMPLES 4 and 5

The following Examples demonstrate the preparation of the superabsorbent polyurethane foam which uses high levels (9.1 and 12.2 pph) of Fibersorb fibers. The control data without superabsorbent was given in the original set of examples.

| | 4 | 5 |
|---|---|---|
| Thanol® SF 5505 polyol | 100 gms | 100 gms |
| L 6202 silicone | 1.1 | 1.1 |
| Amine 1/2/3 catalyst | 0.21 | 0.21 |
| Water | 4.0 | 4.0 |
| T-10 catalyst | 0.4 | 0.4 |
| Propylene carbonate | 10 | 10 |
| Fibersorb (1/8" long) fibers | 15 | 10 |
| TDI | 48.9 | 48.9 |

| Water Absorption Properties | | |
|---|---|---|
| Foam Density, lbs/ft$^3$ | 2.1 | 2.2 |
| Free Swell (g/g) | 25.1 | 23.0 |
| Retention, g/g at 1.2 psi | 13.1 | 14.9 |
| Full Squeeze Retention (g/g) >80 psi | 11.1 | 12.1 |

EXAMPLES 6 and 7

The following examples demonstrate the preparation of the superabsorbent polyurethane foam in the absence of propylene carbonate. A control (Example 6) without the superabsorbent is also shown for comparison. Note all amounts are given in grams. Most water retention properties improve, although the free swell value decreases with the Fibersorb powder addition.

|  | 6 | 7 |
| --- | --- | --- |
| Thanol SF 5505 polyol | 15 gms | 15 gms |
| Thanol F-3550 Polyol | 15 | 15 |
| L 5770 silicone | 0.3 | 0.3 |
| Texcat ZF-51 Catalyst | 0.1 | 0.1 |
| Amine 1/2/3 catalyst | 0.1 | 0.1 |
| Water | 1.2 | 1.2 |
| T-10 catalyst | 0.08 | 0.08 |
| Fibersorb powder | 0 | 4.5 |
| TDI | 15.6 | 15.6 |

<u>Water Retention Properties</u>

| | 6 | 7 |
| --- | --- | --- |
| Free Water Pickup (5 Min.) g/g | 2.0 | 3.0 |
| Free Swell (g/g) | 25.0 | 17.5 |
| Full Squeeze Retention (g/g) | 2.0 | 10.5 |

> 80 PSi

B i K    M

EXAMPLES 8—11

The following examples demonstrate the preparation of superabsorbent polyurethane foam using Fibersorb powder. Note that the control examples without the Fibersorb materials was given in previous Example 1. It will be seen that the water retention properties improve with greater Fibersorb powder levels, and that the foam density remains generally the same for all examples.

|  | 8 | 9 | 10 | 11 |
| --- | --- | --- | --- | --- |
| Thanol SF 5505 polyol | 100 gms | 100 gms | 100 gms | 100 gms |
| L-6202 silicone | 1.1 | 1.1 | 1.1 | 1.1 |
| Amine 1/2/3 catalyst | 0.21 | 0.21 | 0.21 | 0.21 |
| Water | 4.0 | 4.0 | 4.0 | 4.0 |
| T-10 catalyst | 0.4 | 0.4 | 0.4 | 0.4 |
| Fibersorb powder | 5 | 10 | 15 | 20 |
| Propylene carbonate | 10 | 10 | 10 | 10 |
| TDI | 48.9 | 48.9 | 48.9 | 48.9 |

Foam Properties, Water Retention

| | | | | |
|---|---|---|---|---|
| Free Swell (g/g) | 27.9 | 25.1 | 25.0 | 22.8 |
| Retention, g/g at 1.2 psi | 10.1 | 15.2 | 16.0 | 18.0 |
| Full Squeeze Retention (g/g) | 7.4 | 10.9 | 12.4 | 13.5 |
| Foam Density, lbs/ft$^3$ | 2.19 | 2.17 | 2.13 | 2.19 |

EXAMPLES 12 and 13

The following examples demonstrate the preparation of the superabsorbent polyurethane foam in the presence of Freon 11 as a secondary blowing agent. A control without the superabsorbent is also shown for comparison. Note that all amounts are given in grams. It will be appreciated that the water retention properties are greatly increased.

| | 12 | 13 |
|---|---|---|
| Thanol SF 5505 polyol | 15 gms | 15 gms |
| Thanol F-3550 Polyol | 15 | 15 |
| L 5770 silicone | 0.3 | 0.3 |
| Texcat ZF-51 Catalyst | 0.1 | 0.1 |
| Amine 1/2/3 catalyst | 0.1 | 0.1 |
| Water | 1.2 | 1.2 |
| T-10 catalyst | 0.08 | 0.08 |
| Freon 11 | 3.0 | 3.0 |
| Fibersorb powder | 0 | 4.5 |
| TDI | 15.6 | 15.6 |

Water Retention Properties

| | | |
|---|---|---|
| Free Water Pickup (5 Min.) g/g | 2.5 | 6 |
| Free Swell (g/g) | 30.0 | 20.3 |
| Full Squeeze Retention (g/g) | 2.1 | 11.6 |

> 80 PSi

Many modifications may be made in the polyurethanes and polyisocyanurates of this invention and their method of production without departing from the spirit and scope of the invention, which is defined only in the appended claims. For example, one skilled in the art could adjust the temperature, pressure, reactants, proportions and modes of additions within the parameters set forth to provide polyurethane and polyisocyanurate foams with particularly advantageous properties. It is also anticipated that the use of polyethylene oxide polyols would greatly enhance the superabsorbency of the foam within the scope of the invention. It is also anticipated that the foams of this invention would be suitable as an agricultural growing medium, such as a germinating base capable of retaining relatively large quantities of water.

Additionally, the use of polyester polyols as well as various isocyanates, such as, but not limited to MDI (methylene diisocyanate) are also anticipated. It is additionally expected that different forms of the superabsorbent materials, such as fibers and powders may be useful. It is further anticipated that this invention can be used with oil absorbent fibers to produce oil absorbing flexible and rigid foams which are able to retain oil under pressure as well.

8

## GLOSSARY

| | |
|---|---|
| Amine 1/2/3 | Amine catalyst containing 1 part LV33 catalyst (33% triethylenediamine in dipropylene glycol, made by Air Products & Chemicals, Inc.); 2 parts A1 catalyst (dimethyl amine ethyl ether, made by Union Carbide Corp.) and 3 parts polyether polyol. |
| ARSET® catalyst | Reaction product of propylene carbonate and amine catalyst made by ARCO Chemical Company. |
| Fibersorb® polymer | Trade name for a family of organic/inorganic copolymer superabsorbent materials made be ARCO Chemical Company. |
| L6202 Silicone<br>L 5770 Silicone | Silicone surfactants manufactured by Union Carbide Corp. |
| Texcat ZF-51 | Amine catalyst blend containing triethylene diamine, *bis*-dimethylaminoethyl ether and dimethylamino-ethanol in dipropylene glycol, made by Texaco Chemical Co. |
| Thanol® F-3550 polyol | A glycerin initiated polyether of propylene oxide and ethylene oxide modified with the diglycidyl ether of Bisphenol A, with a hydroxyl number of 48 and containing essentially secondary hydroxyl groups, made by ARCO Chemical Company. |
| Thanol® SF 5505 polyol | A glycerin-initiated polyether polyol of propylene oxide and ethylene oxide with a hydroxyl number of 34 and a primary hydroxyl group content of 80% based on the total hydroxyl content of the polyether, made by ARCO Chemical Company. |
| T-10 Catalyst | A 40-50% solution of stannous alkyl hexoate in di-(2-ethylhexyl) phthalate. |

## Claims

1. A superabsorbent foam comprising the product of reacting at least one polyol with at least one polyisocyanate under foam-forming conditions and in the presence of at least one polymeric superabsorbent material.

2. A superabsorbent foam comprising polyurethane or polyisocyanurate foam having dispersed therein polymeric superabsorbent material.

3. A superabsorbent foam as claimed in claim 1 or claim 2 characterised in that said polymeric superabsorbent material comprises an aqueous polymer and a reactive compound.

4. The superabsorbent foam of claim 3 wherein the polymeric superabsorbent material comprises
   (a) an aqueous polymer containing at least 25 mole percent recurring units of an $\alpha,\beta$-unsaturated monomer having in its molecule at least one pendant group selected from carboxyl groups and groups convertible to carboxyl groups, and
   (b) from about 0.1 to about 10 total parts by weight of at least one reactive compound per 100 parts by weight of the aqueous polymer, the reactive compound being a water soluble compound bearing a group selected from amine groups having at least one hydrogen atom attached to a nitrogen atom, hydroxyl groups and mixtures thereof.

5. The superabsorbent foam of claim 4 wherein some but not all of said pendant groups are free carboxylic acid groups and some but not all of said pendant groups are neutralised carboxylic acid groups.

6. The superabsorbent foam of claim 5 wherein the ratio of free carboxylic acid groups to neutralised carboxylic acid groups in the polymer is in the range 1:4 to 4:1 and neutralisation is by means of a base.

7. The superabsorbent foam of any one of claims 4 to 6 wherein the polymer of (a) is derived from maleic anhydride, and the reactive compound is selected from compounds having at least two hydroxyl groups, compounds having at least two amine groups, aminoalcohols and mixtures thereof.

8. The superabsorbent foam of any one of claims 1 to 7 where the proportion of superabsorbent material ranges up to about 25 parts per hundred, based on the total superabsorbent foam, by weight.

9. The superabsorbent foam of any one of claims 1 to 8, where the superabsorbent material is in the form of powder and/or fibre.

10. The superabsorbent foam of any one of claims 1 to 9, obtained from a reaction mixture which additionally includes an alkylene carbonate.

11. The superabsorbent foam of claim 10 where the amount of alkylene carbonate in the reaction mixture ranges from 2 to 50 weight percent.

12. The superabsorbent foam of claim 10 or claim 11 where the alkylene carbonate is propylene carbonate.

13. A method of making a superabsorbent foam as claimed in any one of claims 1 to 12 comprising reacting at least one polyol with at least one polyisocyanate under foam-forming conditions and in the presence of said polymeric superabsorbent material.

14. A method for growing plants employing a superabsorbent foam composition as a growing medium comprising the steps of: in any order:
    (a) introducing water into a growing medium comprising a superabsorbent foam as claimed in any one of claims 1 to 12,
    (b) providing seeds upon or within the growing medium; and
    (c) germinating the seeds upon or within the growing medium

15. The use of a superabsorbent material as claimed in any one of claims 1 to 12 in or as a growing medium for growing plants.